# EUROPEAN PATENT APPLICATION

(11) **EP 0 566 971 A1**
(43) Date of publication of application: **27.10.1993**
(21) Application number: 93106026.3
(22) Date of filing: 14.04.1993
(51) Int. Cl.: A61K 31/205

(54) **Use of L-carnitine for the treatment of shock**

(30) Priority: 23.04.1992 IT RM920305
(71) Applicant: Sigma-Tau Industrie Farmaceutiche Riunite S.p.A., I-00144 Roma (IT)
(72) Inventor: Cavazza, Claudio, Dr., I-00186 Roma (IT)
(74) Representative: La Ciura, Salvatore

(57) **Abstract**

The intravenous administration if a bolus of 3-5 grams L-carnitine or a pharmacologically acceptable salt thereof followed by 3-5 grams of L-carnitine or salt thereof vis slow infusion in about 12 hours, dramatically restore to normal the arterial pressure and respiratory frequency in a patient exhibiting serious cardiogenic or septic shock conditions.

## Description

The present invention relates to a novel therapeutic utilization of L-carnitine and its pharmacologically acceptable salts of the therapeutic of cardiogenic and septic shock.

Previous therapeutical uses of L-carnitine are already known.

For instance, L-carnitine has been used in the cardiovascular field in the treatment of acute and chronic myocardial ischaemia, angina pectoris, cardiac arrhythmias and insufficiency. In nephrology, L-carnitine has been administered to chronic uraemic patients who are subjected to regular haemodialysis treatment with a view to counteracting muscular asthenia and the onset of muscular cramps. Further therapeutical uses are the restoration of the HDL/LDL+VLDL ratio to normal and in total parenteral nutrition. The use of L-carnitine for the treatment of certain myopathies and muscular dystrophies is also known.

However, there is no relationship between the foregoing known therapeutical utilizations of L-carnitine and the utilization which is the object of the present invention.

It is well-known that shock is as clinical condition essentially characterized by an insufficient tissue perfusion, with usually severe hypotension which, if untreated, is generally fatal. Shock may be brought about by different causes, such as serious hemorrhages, cranial trauma, dangerous cardiac insufficiency as in certain myocardial infarctions and anaphylactic reactions.

Therapy at present in actual use is not suited for all kinds of shock conditions and frequently turns out to be unsatisfactory.

Generally, in all shock conditions, the trend is to restore blood volume by means of blood, plasma, saline or glucose solutions or plasma substituents infusion, and to administer oxygen.

However, in severe shock conditions, said treatment is usually insufficient if not even counteracting. In fact, in cardiogenic shock, infusion of liquids would overload the heart, whose function is already seriously impaired because of the inadequate myocardial contractility.

Vasoconstrictor drugs, such as noradrenaline, adrenaline, metaraminol, mephentermine, which are administered in order to increase pressure, often bring about the opposite effect, since, in shock conditions (barring neurogenic shock) a severe sympathetic reflex vasoconstriction is present already, whereby tissue perfusion would be further impaired.

Therefore, administration of drugs such as dopamine, dobutamine, isoproterenol and glucagon, which improve cardiac inotropism without substantially increasing the peripheral resistances, is preferred, particularly in case of cardiogenic shock.

On the other hand, in some instances, administration of vasodilator drugs such as nitroprussiate and alpha-blockers may be convenient, in order to improve tissue perfusion.

Although cortiscosteroids are widely used in the treatment of shock, no convincing proof is available Which could support the effectiveness of said drugs.

Recently, the effectivenes's of naloxone in different models of shock has been studied. Although naloxone turned out to be effective in restoring blood pressure to normal values, it is absolutely contra-indicated in overdose-induced shock. It is in fact known that naloxone administration to drug addicts is followed by a typical abstinence syndrome.

Now it has surprisingly been found that the use of L-carnitine and its pharmacologically acceptable salts is dramatically effective in the therapeutic treatment of shock, particularly of cardiogenic and septic shock.

Therefore, in accordance with the present invention, L-carnitine and its pharmacologically acceptable salts are used for producing a pharmaceutical composition effective for the therapeutical treatment of such shock conditions.

In practice, a patient in a state of shock is administered a bolus of 3-5 gram of L-carnitine. Preferably, following the administration of the L-carnitine bolus, the patient is further administered 3-5 grams of L-carnitine or an equivalent amount of a pharmacologically acceptable salt via slow infusion over a period of about 12 hours.

It is apparent that in the light of the therapeutical use according to the present invention the most suitable pharmaceutical compositions are those compositions which are apt to be administered via the parenteral route.

Hereinbelow, the results of a clinical trial are shown; the results are expressed as dead patients/treated patients ratio both in the control group and in the group of patients treated according to the invention.

The admission criteria were as follows:
(1) systolic pressure lower than 90 mmHg or lower than 30% of patint's normal pressure;
(2) urine volume lower than 20 ml/hour;
(3) severe metabolic acidosis;
(4) weakened sensorial functions.

Each patient of both the control group and treated group received a basal treatment as hereinbelow indicated. The controls received the basal treatment only.

| Therapy | Septic shock | Cardiogenic shock |
|---|---|---|
| Alkalizers | NaHCO₃ till adjustment of two thirds of electrolytic balance | NaHCO₃ till adjustment of two thirds of electrolytic balance |
| Antacids | Cimetidine, 1 vial i.v. x 4 | Cimetidine, 1 vial i.v. x 4 |
| Antibiotics | According to the bacteria involved | Not considered |
| Sedative and analgesics | On physician's advice | On physician's advice |
| Corticosteroids | Solu-Medrol, 30 mg/kg i.v. q. 6 hrs (for the first 12 hrs) | Not considered |
| Diuretics | Mannitol, 25 g on admission and q. 12 hrs thereafter | Furosemide, 40-60 mg i.v. following haemodynamic evaluation |
| | Fast volumetric restitution with blood, dextrane and Ringer lactate | Volumetric restitution according to Gunnar and Loeb |
| Vasoactive drigs | According to haemodynamic evaluation | According to haemodynamic evaluation |
| Cardiotonics | Cedilanid, 1 vial i.v. q. 12 hrs | Not considered |

The clinical trial results were as follows:

| | dead/treated | |
|---|---|---|
| | cardiogenic shock | septic shock |
| Controls | 8/8 | 5/5 |
| L-carnitine bolus 4 g, i.v. + 4 g by infusion in 12 hours | 2/13 | 0/2 |

Venous blood samples were drawn from all the patients upon their admission to the intensive care unit and at 3-hours intervals during the subsequent 12 hours. The last samples were not drawn from the control group patients because of patient's death. Succinate and fumarate levels were determined in plasma samples by the gas-liquid chromatography procedure discolsed by Chazmers et al. in "Quantitative extraction and gas liquid chromatographic determination of organic acids in plasma", Analyst (1972) 97, 958-967.

The untreated patients showed a progressively increase in plasma succinate and an attendant decrease in fumarate. Conversely, the L-carnitine - treated patients showed a progressively diminishing succinate concentration and steady increase in fumarate.

## Claims

1. Use of L-carnitine and pharmacologically acceptable salts tehereof for the therapeutic treatment of shock conditions, particularly cardiogenic or septic shock.

2. Use of L-carnitine according to the preceding claim comprising the i.v. administration to a shock patient of 3-5 grams L-carnitine bolus or an equivalent amount of a pharmacologically acceptable salt thereof.

3. Use of L-carnitine or salts thereof according to the preceding claim which comprises, following the administration of L-carnitine bolus, the administration of further 3-5 grams L-carnitine in about 12 hours by slow infusion.

4. Use of L-carnitine and the pharmacologically acceptable salts thereof for manufaturing a pharmaceutical composition for the therapeutical treatment of shock conditions.

5. A parenterally administrable pharmaceutical composition in unite dosage form for use according to claim 2, comprising from about 500 to about 1,500 mg L-carnitine and a pharmacologically acceptable excipient thereof.

6. The parenterally administrable pharmaceutical composition of claim 5, as an injectable phial.
